# EUROPEAN PATENT SPECIFICATION

(11) **EP 3 830 067 B1**
(45) Date of publication and mention of the grant of the patent: **26.10.2022**
(21) Application number: 19745645.2
(22) Date of filing: 02.08.2019
(51) Int. Cl.: C07C 51/16, C07C 53/126, C11C 1/02, C11C 3/00, C07C 51/29, C08L 91/00, C11B 11/00, C07C 53/128

(54) **PROCESS FOR THE CLEAVAGE AND OXIDATION OF ESTERS**
VERFAHREN ZUR SPALTUNG UND OXIDIERUNG VON ESTERN
PROCÉDÉ DE CLIVAGE ET D'OXYDATION D'ESTERS

(30) Priority: 03.08.2018 EP 18187220; 03.08.2018 EP 18187234
(43) Date of publication of application: 09.06.2021
(73) Proprietor: Völpker Spezialprodukte GmbH, 39393 Völpke (DE); Kahl GmbH & Co. KG, 22946 Trittau (DE)
(72) Inventor: HOFMANN, Tommy, 56379 Horhausen (DE); KÜHN, Fritz Elmar, 85748 Garching (DE); NAGORNY, Nicolai, 22949 Ammersbek (DE); SCHLÜTER, Marc, 22339 Hamburg (DE)
(74) Representative: Sandvoß, Stefanie
(86) International application number: PCT/EP2019/070937
(87) International publication number: WO 2020/025813

(56) References cited:
- EP-A1- 1 149 846
- US-A- 5 166 422
- US-A- 5 387 712
- US-A- 6 031 101
- US-A1- 2015 284 661

## Description

The present invention provides a process for the oxidation of long chain alcohols to carboxylic acids under environmentally acceptable and economically advantageous conditions.

The oxidation of long chain alcohols to provide the corresponding carboxylic acids plays a relevant role for the refining of waxes and wax hydrolysates (Ullmann's Encyclopedia of Industrial Chemistry, "Waxes", Vol. 39, 6th ed., Weinheim, 2003, p. 135). The oxidation of waxes (and wax hydrolysates), e.g. montan wax (MW) or rice bran wax (RBW), is carried out on an industrial scale with more than 10,000 tons of raw material per year.

Currently, long chain alcohols are often either molten and oxidized with chromosulfuric acid, a mixture of Cr(VI) reagents and sulfuric acid (DE 102 31 886 A1, WO 2014/060081 A1), or are oxidized at lower temperatures as a solution in an organic solvent. However, the use of toxic Cr(VI) reagents in large quantities is environmentally critical. To date, of about 25,000 t montan wax, about 60% are oxidized using chromosulfuric acid. If organic solvents are used, the recovery of the oxidized product from the solution or the removal of the solvent from the product are required.

US 5387712 relates to a process for the preparation of an aromatic alkoxyalkanoic acid by reacting the corresponding alcohol with a stable free radical nitroxide in the presence of a NOₓ-generating compound and an oxidant. US 5166422 discloses a process for the preparation of an alkanoic acid which comprises the reaction of an alkanol with a solubilized stable free radical nitroxide. US 6031101 relates to the preparation of an endothelin antagonist which comprises the oxidation of an intermediate bearing a primary alcohol using sodium chlorite in the presence of a catalytic amount of TEMPO and sodium hypochlorite. EP 1149846 A1 relates to the oxidation of polysaccharides in an aqueous solution or suspension using hypochlorite in the presence of nitroxyls.

The present invention provides a process which allows the oxidation of long chain alcohols or compositions comprising such alcohols to be achieved while not requiring the use of organic solvents or of reagents which are very harmful to the environment, such as heavy metals.

Thus, the invention relates to a process for the oxidation of alcohols, in particular alcohols with a carbon number of 14 or more, to carboxylic acids, wherein said process comprises:
a step of subjecting a starting material comprising one or more primary alcohols with a carbon number of 14 or more to an oxidation reaction using an oxoammonium cation and a co-oxidant as oxidizing agents at a reaction temperature of 50°C or higher to obtain one or more carboxylic acids.

It has been found that the process of the present invention allows the oxidation of long chain alcohols to be achieved under environmentally acceptable and mild conditions with high yields and without undesirable side reactions, such as the formation of esters or a cleavage of carbon-carbon bonds in the alcohols subjected to the oxidation reaction.

Oxidations wherein the oxoammonium cation acts as an oxidizing catalyst, are known to proceed in organic solvents or with reactants which are soluble in water (cf. R. Ciriminna, M. Pagliaro, Organic Process Research & Development, 2010, 14, 245-251). The oxoammonium cation can be generated *in situ* using aminoxyl radicals (also referred to as N-oxyl radicals, nitroxyl radicals or nitroxides) or the corresponding hydroxylamines as reactants. It was found in the context of the present invention that oxoammonium mediated oxidations can be successfully employed for higher alcohols showing a very limited or no solubility in water, yet without the need to employ additional organic solvents.

The starting material that is subjected to the oxidation reaction in the context of the process of the present invention comprises one or more primary alcohols with a carbon number of 14 or more. As will be understood by the skilled reader, the reference to an alcohol herein relates to an organic compound carrying a hydroxy group -OH bound to a saturated carbon atom. Accordingly, a primary alcohol is an organic compound wherein the hydroxy group is bound to a carbon atom which, in turn, is bound to only one further carbon atom, and to two hydrogen atoms. In other words, the primary alcohol is an organic compound which comprises a structural element of the formula R-CH₂-OH, wherein R is an organic residue bound via a carbon atom to the -CH₂-OH moiety. The respective hydroxy group is also referred to as primary hydroxy group or primary alcohol group.

The oxidation reaction to which the starting material comprising one or more primary alcohols with a carbon number of 14 or more is subjected during the process of the present invention leads to the oxidation of primary alcohols to carboxylic acids. As shall be explained herein in further detail, the one or more primary alcohols with a carbon number of 14 or more may be quantitatively (i.e. completely) oxidized to carboxylic acids, or, if desired, the oxidation reaction may be controlled such that only a part of the primary alcohols is oxidized. In that case, the material resulting from the oxidation reaction will still contain a part of the primary alcohols that are contained in the starting material.

The starting material for the oxidation reaction may contain further alcohols in addition to the one or more primary alcohols with a carbon number of 14 or more, i.e. alcohols with a lower carbon number. However, in the starting material used in the process of the present invention such alcohols with a carbon number of less than 14 are often not present in any significant amounts, or may be completely absent, so that the starting material may be a starting material wherein no further alcohols apart from the one or more primary alcohols with a carbon number of 14 or more are contained.

The one or more primary alcohols with a carbon number of 14 or more may comprise in their molecular structure further functional groups containing one or more heteroatoms, including additional hydroxy groups, together with the primary hydroxy group. However, preferably the one or more primary alcohols with a carbon number of 14 or more comprise one primary alcohol function, and no further functional groups.

The process in accordance with the present invention is preferably used for the oxidation of one or more aliphatic primary alcohols with a carbon number of 14 or more, and even more preferably of one or more alcohols of the formula H₃C-(CH₂)ₙ-OH, wherein n+1 is an integer which corresponds to the carbon number of the alcohol, i.e. n + 1 is 14 or more (or n is an integer of 13 or more). Thus, the starting material subjected to the oxidation reaction preferably comprises one or more aliphatic primary alcohols with a carbon number of 14 or more, and even more preferably of one or more alcohols of the formula H₃C-(CH₂)ₙ-OH, wherein n+1 is an integer which corresponds to the carbon number of the alcohol, i.e. n + 1 is 14 or more.

As will be understood by the skilled reader, the carbon number as referred to herein indicates the total number of carbon atoms in an organic compound, i.e. in the present case the total number of carbon atoms contained in the molecule of the primary alcohol.

The carbon number of the one or more primary alcohols is 14 or more. Preferably, the starting material comprises one or more primary alcohols having a carbon number of 16 or more. More preferably, the starting material comprises one or more primary alcohols selected from primary alcohols with a carbon number of 16 or more and 42 or less, more preferably 20 or more and 40 or less. It will be understood that these preferred values are also to be considered as preferred values for n+1 in the formula H₃C-(CH₂)ₙ-OH as discussed above.

The starting material may comprise a mixture of primary alcohols with different carbon numbers.

Thus, in view of the above, it will be understood that, in accordance with a particularly preferred embodiment, the starting material subjected to the oxidation reaction comprises one or more primary alcohols of the formula H₃C-(CH₂)ₙ-OH, wherein n + 1 is an integer and is 16 or more (i.e. n is an integer of 15 or more). More preferably, n+1 is 16 or more and 42 or less (i.e. n is 15 or more and 41 or less), still more preferably n+1 is 20 or more and 40 or less (i.e. n is 19 or more and 39 or less). The starting material may be free of alcohols other than these preferred or more preferred alcohols.

The starting material subjected to the oxidation reaction may contain other organic components apart from the one or more primary alcohols discussed above.

According to the invention, the starting material comprising the one or more primary alcohols discussed above that is subjected to the oxidation reaction in the context of the process of the present invention is a wax, preferably a natural wax, or may be derived from a wax, preferably from a natural wax. Starting materials derived from a wax include in particular a material which is isolated from a wax, or a material which is obtained by processing a wax, e.g. by fully or partially hydrolyzing esters contained therein. Such a material obtained by fully or partially hydrolyzing esters contained in a wax is also referred to as a hydrolyzed wax or a split wax. Natural waxes, including fossil waxes such a montan wax and non-fossil waxes, such as rice bran wax, sunflower wax, carnauba wax, candelilla wax, sugarcane wax and beeswax, typically contain free primary alcohols with a carbon number of 14 or more, and esters formed from carboxylic acids and such alcohols. Increasing the acid number of the waxes via oxidation of the free alcohols or via hydrolysis of wax esters, and oxidation of the alcohols that are formed during the hydrolysis may improve the wax characteristics, such as processability, or their suitability for certain applications. Thus, the starting material subjected to the oxidation reaction which comprises the alcohols discussed above is a wax or a processed wax resulting from a process comprising a full or partial hydrolysis of the wax esters that had been contained in the wax before processing, and preferably a natural wax or a processed natural wax resulting from process comprising a full or partial hydrolysis of the wax esters that had been contained in the natural wax before processing.

It will be understood that the information provided above with respect to the preferred type of the primary alcohol in terms of its carbon number or its structure also applies for the primary alcohol comprised in the wax or in the processed wax.

Thus, the process of the present invention may be used to oxidize one or more primary alcohols with a carbon number of 14 or more contained in a wax, or it may be used to oxidize one or more primary alcohols with a carbon number of 14 or more in a processed wax obtained by full or partial hydrolysis of esters contained in a (unprocessed) wax. To that extent, the process of the present invention may comprise, prior to the step of subjecting the starting material to the oxidation reaction, a preliminary step of providing the starting material for the oxidation reaction by processing a wax, preferably a natural wax, by a process comprising fully or partially hydrolyzing esters contained therein, and using the processed wax, preferably the processed natural wax, as a the starting material for the oxidation reaction.

If a processed wax is used as a starting material, the processed wax may be used for the oxidation reaction directly after the full or partial hydrolysis of the esters, or may be subjected to a work-up procedure if desired.

The starting material comprising the one or more primary alcohols discussed above is subjected in the process of the invention to an oxidation reaction using an oxoammonium cation and a co-oxidant as oxidizing agents.

As will be understood be the skilled reader, the oxidation reaction may be accomplished using one single type of an oxoammonium cation, or two or more types of oxoammonium cations differing in their structure. Similarly, the oxidation reaction may be accomplished using one single type of co-oxidant, or two or more types of co-oxidants differing in their structure.

As noted above, the use of oxoammonium cations as oxidation catalysts is known in the art (e.g. R. Ciriminna et al., Organic Process Research & Development, 2010, 14, 245-251 or M. Shibuya et al., J. Am. Chem. Soc., 2006, 128, 8412-8413), and the oxoammonium cation may be generated in situ for this purpose using an corresponding aminoxyl radical or hydroxylamine as a precursor compound.

Suitable oxoammonium cations which can also be employed in the process in accordance with the invention typically have a structure which can be schematically represented as R₂N⁺=O, wherein the two groups R are independently an organic group bound via a secondary or tertiary carbon atom (i.e. a carbon atom bound to two or three further carbon atoms, respectively) to the nitrogen, and wherein the two groups R may be combined to form a heterocyclic ring together with the nitrogen atom to which they are bound. The anion which acts as a counter-ion for the cationic oxoammonium ion is not particularly limited. Examples are halide ions, such as chloride or bromide, and hydroxy ions, and preferred are chloride or bromide.

Preferred as oxoammonium cation for use in the process in accordance with the invention is an oxoammonium cation selected from a 2,2,6,6-tetramethylpiperidine-oxoammonium cation and from derivatives thereof carrying a substituent at the 4-position of the piperidine ring, from a 9-azabicyclo[3.3.1]nonane-oxoammonium cation, and from a 2-azaadamantane-oxoammonium cation, a 1-methyl-2-azaadamantane-oxoammonium cation and a 1,3-dimethyl-2-azaadamantane-oxoammonium cation.

Thus, a further preferred oxoammonium cation for use in the process in accordance with the invention is an oxoammonium cation having a structure as shown in the following formulae (la) to (Ilia):

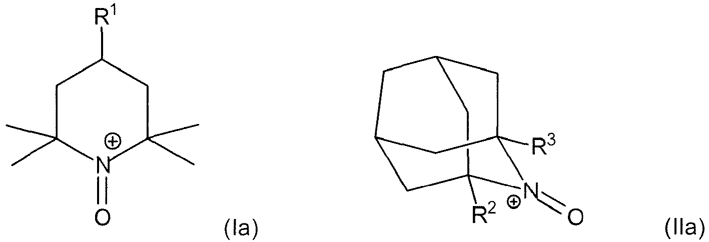

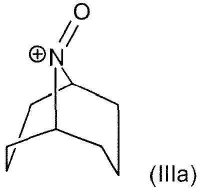

In formula (la), R¹ is selected from hydrogen, -OH, =0, -OR⁴, -NH₂, and -NHC(O)R⁵. R⁴ designates an alkyl group, an aryl group, an aralkyl group or a group -C(O)R⁶, with R⁶ being selected from a C1-C6 alkyl group and a phenyl group. The alkyl group is preferably a C1-C6 alkyl group, the aryl group is preferably a phenyl group and the aralkyl group is preferably a benzyl group. More preferably, R⁴ designates a C1-C6 alkyl group, and most preferably a methyl group. R⁵ designates a C1-C6 alkyl group, and preferably a methyl group. It will be understood that if R¹ is =O, the carbon atom to which R¹ is attached carries no hydrogen atom.

In formula (IIa), R² and R³ are independently selected from hydrogen and methyl.

The oxoammonium cation can be added to the reaction mixture in the form of the oxoammonium salt, or can be generated *in situ,* i.e. in the reaction mixture for the oxidation reaction, from a suitable precursor compound, for example from an aminoxyl radical or from a hydroxylamine. Preferably, the precursor compound is an aminoxyl radical. Thus, the process of the present invention may comprise:
a step of providing a reaction mixture comprising (i) the starting material comprising the one or more primary alcohols as discussed above, (ii) a precursor compound for an oxoammonium cation, and (iii) a co-oxidant, and
allowing the precursor compound to form an oxoammonium cation.

As will be understood by the skilled reader, the reaction mixture as referred to above would be provided and the oxoammonium cation would be allowed to form before the starting material comprising the alcohol is subjected to the oxidation reaction.

In order to provide an oxoammonium cation as discussed above, an aminoxyl radical which may be used as a precursor compound in the process of the present invention is typically an aminoxyl radical having the structure R₂N-O•, wherein the dot at the oxygen atom indicates an unpaired electron, and wherein the two groups R are independently an organic group bound via a secondary or tertiary carbon atom to the nitrogen, and wherein the two groups R may form a heterocyclic ring together with the nitrogen atom to which they are bound.

More preferably, the aminoxyl radical for use in the process of the present invention is selected from 2,2,6,6-tetramethylpiperidine-1-oxyl (TEMPO) and from derivatives thereof carrying a substituent at the 4-position of the piperidine ring, from 9-azabicyclo[3.3.1]nonane N-oxyl (ABNO), and from 2-azaadamantane N-oxyl (AZADO), 1-methyl-2-azaadamantane N-oxyl (1-Me-AZADO) and 1,3-dimethyl-2-azaadamantane N-oxyl (1,3-dimethyl-AZADO).

As suitable 4-substitued TEMPO derivatives, the following exemplary compounds can be specifically mentioned for use in the context of the present invention: 4-OH-TEMPO, 4-Oxo-TEMPO, 4-methoxy-TEMPO (4-MeO-TEMPO), 4-NH₂-TEMPO, 4-acetamido-TEMPO (4-AA-TEMPO), 4-carboxy-TEMPO, and 4-hydroxy-TEMPO benzoate.

Thus, a further preferred aminoxyl radical for use as a precursor compound in the process in accordance with the invention is an aminoxyl radical having a structure as shown in the following formulae (Ib) to (IIIb):

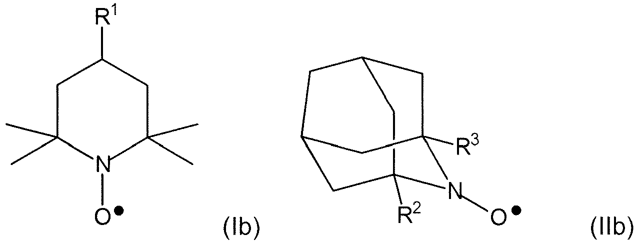

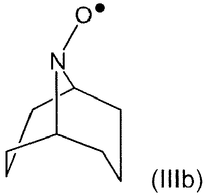

In formula (lb), R¹ is selected from hydrogen, -OH, =O, -OR⁴, -NH₂, and -NHC(O)R⁵. R⁴ designates an alkyl group, an aryl group, an aralkyl group or a group -C(O)R⁶, with R⁶ being selected from a C1-C6 alkyl group and a phenyl group. The alkyl group is preferably a C1-C6 alkyl group, the aryl group is preferably a phenyl group and the aralkyl group is preferably a benzyl group. More preferably, R⁴ designates a C1-C6 alkyl group, and most preferably a methyl group. R⁵ designates a C1-C6 alkyl group, and preferably a methyl group. It will be understood that if R¹ is =0, the carbon atom to which R¹ is attached carries no hydrogen atom.

In formula (lib), R² and R³ are independently selected from hydrogen and methyl.

The aminoxyl radical as discussed above may also be used in the process in accordance with the invention in immobilized form on a carrier, e.g. supported by a carrier without chemical bonds between the carrier and the aminoxyl radical, or covalently bound to a carrier. Preferred carriers are a silica gel or a polymer. In this regard, TEMPO and its 4-substituted derivatives, such as 4-OH-TEMPO and 4-NH₂-TEMPO are particularly useful. The substituent in 4-position may act as a functional group for the formation of a bond with the carrier (e.g. an ether bond, an ester bond, an amino bond or an amide bond).

Particularly preferred as aminoxyl radicals for use in the context of the present invention are 4-OH-TEMPO, 4-NH₂-TEMPO, 4-MeO-TEMPO, 4-AA-TEMPO, TEMPO, Me-AZADO, ABNO, or any of these aminoxyl radicals supported on or bound to a carrier. Most preferred in terms of a balance between costs and efficiency is 4-OH-TEMPO as aminoxyl radical.

An aminoxyl radical can be conveniently used as a precursor compound of an oxoammonium cation, since aminoxyl radical compounds are commercially available and can form an oxoammonium cation spontaneously via disproportionation of the radical. However, the formation of the cation may also be accelerated by adding a component for the acceleration of the reaction as discussed below.

As far as examples of hydroxylamines are concerned which are suitable as precursor compounds in the process in accordance with the invention, the above information regarding the general and preferred structures of suitable aminoxyl radicals equally applies, except for the fact that the hydroxylamine carries an -OH or -OR substituent attached to the nitrogen atom instead of a radical -O•. In these hydroxylamines, R is a hydrocarbon group such as an alkyl group, an aryl group or an aralkyl group. The alkyl group is preferably a C1-C6 alkyl group, the aryl group is preferably a phenyl group and the aralkyl group is preferably a benzyl group. However, if a hydroxylamine is used as as a precursor compound of an oxoammonium cation, the hydroxylamine is preferably a compound which carries an -OH substituent instead of a radical -O• attached to the nitrogen atom of the aminoxyl radicals and preferred aminoxal radicals discussed above.

Without wishing to be bound by theory, it is assumed that the oxoammonium cation can act as a catalytically active species in the oxidation reaction. In this reaction, the oxoammonium cation is reduced to a hydroxylamine while the primary alcohol is oxidized. In the presence of a co-oxidant which can oxidize the hydroxylamine, the oxoammonium cation can be recovered.

In the process of the present invention, an oxoammonium cation or a precursor thereof, such as an aminoxyl radical or a hydroxylamine, is typically combined with the starting material comprising the alcohol in order to subject it to the oxidation reaction in an amount of 0.01 mol% or more, preferably 0.1 mol% or more, more preferably 0.2 mol% or more, and still more preferably 0.5 mol% or more, based on the total number of moles of primary alcohol group in the starting material. When the amount of oxoammonium cation or precursor thereof is increased, the yield of carboxylic acid and/or the rate of the reaction may increase. However, when a certain amount is exceeded, no further improvements in terms of an increased yield or reaction rate are observed. Moreover, the costs of the process are higher with a higher amount of aminoxyl radical. Thus, the oxoammonium cation or a precursor thereof is typically combined with the starting material in the process in accordance with the invention in amounts of not more than 20 mol%, preferably not more than 10 mol%, more preferably not more than 5 mol%, and still more preferably not more than 3 mol%, based on the total number of moles of primary alcohol group in the starting material.

A further component which is used in the process in accordance with the invention is the cooxidant. An effective co-oxidant for use in the process of the present invention is preferably selected from a chlorite (i.e. a salt with the anion ClO₂⁻) and a bromite (i.e. a salt with the anion BrO₂⁻). More preferably, a chlorite is used.

Suitable cations for the chlorite and the bromite include, e.g., an ammonium cation or alkali cations such as Na⁺ and K⁺. In terms of costs and availability, sodium chlorite is a preferred co-oxidant.

In the process of the present invention, the amount of the co-oxidant which is combined with the starting material comprising the one or more primary alcohols in order to subject it to the oxidation reaction can be suitably chosen depending on the desired rate of the oxidation reaction. For example, if only a part of the primary alcohols is to be oxidized, an amount of the co-oxidant of less than 1 molar equivalent (eq.) of co-oxidant per mole of primary alcohol groups in the starting material can be used, e.g. 0.75 eq. For a complete oxidation, the cooxidant should be used in an amount of 1.0 molar equivalents or more, preferably in a slight excess compared to the primary alcohol group to be oxidized. Thus, the co-oxidant is preferably used in an amount of 0.75 molar equivalents or more, more preferably 1.0 molar equivalents or more, even more preferably 1.05 molar equivalents or more, per mole of primary alcohol group in the starting material. The maximum amount is not particularly limited, but amounts of more than 1.5 molar equivalents in general do not give rise to advantages and increase the costs of the reaction. Thus, the co-oxidant is preferably used in an amount of 1.5 molar equivalents or less, more preferably 1.3 molar equivalents or less, per mole of primary alcohol group in the starting material.

Especially in cases where a precursor compound of an oxoammonium cation, such an an aminoxyl radical, is combined with the starting material comprising the one or more primary alcohols , the start of the oxidation reaction to which the starting material comprising the one or more primary alcohols is subjected may be accelerated by additionally adding a component selected from a hypochlorite (i.e. a salt with the anion OCI⁻), a hypobromite (i.e. a salt with the anion OBr⁻), chlorine gas or any chemical that in-situ produces one of these substances, e.g., trichloroisocyanuric acid, an aldehyde in combination with NaClO₂. Among these, hypochlorite and hypobromite are preferred, and the hypochlorite is particularly preferred. Suitable cations for the hypochlorite and the hypobromite include, e.g., an ammonium cation or alkali cations such as Na⁺ and K⁺. In terms of costs and availability, sodium hypochlorite is a preferred optional component for the acceleration of the reaction.

If such a component for the acceleration of the reaction is used, it is typically used in amounts of 0.2-5.0 mol% per mole of primary alcohol group in the starting material comprising the one or more primary alcohols. Moreover, it is preferred that the amount of the component for the acceleration of the reaction is used in a molar ratio in the range of 1-2 with regard to the amount of the precursor compound of the oxoammonium cation.

In view of the above, it will be understood that in accordance with a particularly preferred embodiment, the process in accordance with the present invention comprises
a step of providing a reaction mixture comprising (i) the starting material comprising the one or more primary alcohols, (ii) an aminoxyl radical as a precursor compound for an oxoammonium cation and (iii) a co-oxidant,
allowing the precursor compound to form an oxoammonium cation, and a step of subjecting the starting material to an oxidation reaction using the oxoammonium cation and the co-oxidant as oxidizing agents at a reaction temperature of 50°C or higher, wherein
the starting material comprises one or more primary alcohols of the formula H₃C-(CH₂)ₙ-OH, wherein n + 1 is an integer and is 16 or more, and more preferably, n+1 is 16 or more and 42 or less, still more preferably 20 or more and 40 or less,
the aminoxyl radical is an aminoxyl radical having the structure R₂N-O•, wherein the dot at the oxygen atom indicates an unpaired electron, and wherein the two groups R are independently an organic group bound via a secondary or tertiary carbon atom to the nitrogen, and wherein the two groups R form a heterocyclic ring together with the nitrogen atom to which they are bound, and is more preferably selected from 4-OH-TEMPO, 4-NH₂-TEMPO, 4-MeO-TEMPO, 4-AA-TEMPO, TEMPO, Me-AZADO, ABNO, and from any of these aminoxyl radicals supported on or bound to a carrier,
the co-oxidant is a chlorite,
and optionally a hypochlorite is additionally added prior to the oxidation reaction to which the starting material is subjected.

As noted above, in order to carry out the process in accordance with the present invention, the use of organic solvents is not necessary. Thus, the process is preferably carried out in the absence of an organic solvent. In this regard, it will be understood that a solvent, as referred to herein, is a substance which is liquid below the melting point of the starting material comprising the one or more primary alcohols and atmospheric pressure (e.g. 1013.25 hPa) and which is used in a reaction to dissolve or disperse one or more of the reactants. In other words, the starting material comprising the one or more primary alcohols, which is typically a solid or a soft solid at room temperature and atmospheric pressure is obviously not encompassed by the organic solvents as referred to above. The melting point of the starting material comprising the one or more primary alcohols is generally the lowest temperature at which the starting material can form a liquid phase.

It will further be understood that the oxidation reaction is typically carried out in the absence of transition metals and transition metal compounds, such as chromium reagents.

On the other hand, the oxidation reaction using an oxoammonium cation and a co-oxidant is preferably carried out in the presence of an aqueous medium. As will be understood by the skilled reader, an aqueous medium is a medium formed by water as a solvent, wherein one or more components may be dissolved or dispersed. In the preferred case where an aqueous medium is present during the oxidation reaction in the process according to the invention, a significant portion of the oxoammonium cation and of the co-oxidant acting as oxidizing agents can be contained in the aqueous medium. In order to provide the reaction mixture for the oxidation reaction, the oxoammonium cation or a precursor compound thereof, the co-oxidant and the optional component for the acceleration of the reaction may, e.g., be dissolved in one or more aqueous media prior to the oxidation reaction and added to the starting material including the alcohols or to a mixture of the starting material and an aqueous medium prior to the oxidation reaction.

Moreover, in the preferred case where an aqueous medium is present during the oxidation reaction in the process according to the invention, the aqueous medium is preferably adjusted to a pH of 8 or less, more preferably a pH of 6 or less. Typically, an acid or a buffer is present in the aqueous medium in order to adjust the pH thereof. Exemplary suitable acids include HCI, H₃PO₄, citric acid and acetic acid. Exemplary suitable buffers include an acetic acid/acetate buffer.

In the preferred case where an aqueous medium is present during the oxidation reaction in the process according to the invention, the aqueous medium may form a separate phase in the reaction mixture of the oxidation reaction in view of the fact that the primary alcohols with a carbon number of 14 or more typically show hydrophobic characteristics. Depending on the relative volume ratio of the aqueous medium and of the organic phase comprising the starting material and/or the carboxylic acids which are formed during the oxidation reaction, the aqueous medium may form a continuous phase during the oxidation reaction wherein the organic phase is dispersed, or the organic phase may form a continuous phase wherein the aqueous phase is dispersed.

The volume ratio of the organic phase comprising the starting material to be subjected to the oxidation reaction to the aqueous medium is not particularly limited. Typical volume ratios range from 10:1, preferably 5:1, more preferably 3:2, to 1:10, preferably 1:5 (indicated as volume organic phase:volume aqueous medium at the reaction temperature of the oxidation reaction and at the start of the oxidation reaction).

Thus, in line with a particularly preferred embodiment, the process in accordance with the present invention comprises
a step of providing a reaction mixture comprising (i) the starting material comprising the one or more primary alcohols, (ii) an aminoxyl radical as a precursor compound for an oxoammonium cation, (iii) a co-oxidant, and (iv) an aqueous medium,
allowing the precursor compound to form an oxoammonium cation, and a step of subjecting the starting material to an oxidation reaction using the oxoammonium cation and the co-oxidant as oxidizing agents at a reaction temperature of 50°C or higher, wherein
the starting material comprises one or more primary alcohols of the formula H₃C-(CH₂)ₙ-OH, wherein n + 1 is an integer and is 16 or more, and more preferably, n+1 is 16 or more and 42 or less, still more preferably 20 or more and 40 or less,
the aminoxyl radical is an aminoxyl radical having the structure R₂N-O•, wherein the dot at the oxygen atom indicates an unpaired electron, and wherein the two groups R are independently an organic group bound via a secondary or tertiary carbon atom to the nitrogen, and wherein the two groups R form a heterocyclic ring together with the nitrogen atom to which they are bound, and is more preferably selected from 4-OH-TEMPO, 4-NH₂-TEMPO, 4-MeO-TEMPO, 4-AA-TEMPO, TEMPO, Me-AZADO, ABNO, and from any of these aminoxyl radicals supported on or bound to a carrier,
the co-oxidant is a chlorite,
and optionally a hypochlorite is additionally added prior to the oxidation reaction to which the starting material is subjected.

The reaction temperature of the oxidation reaction is 50°C or higher. Usually, the temperature is adjusted such that the starting material is softened or molten, preferably molten, so that the reactants forming the reaction mixture can be conveniently mixed. Thus, the oxidation reaction is preferably carried out at a temperature where the starting material for the oxidation reaction is in a liquid form (i.e. a molten liquid form). This temperature may be a temperature higher than the melting points of the individual components in the starting material. However, since a mixture of organic compounds may have a melting point which is lower than the individual melting points of the components of the mixture, a molten liquid may already exist at a temperature which is lower than the lowest individual melting point of the components of the starting material.

Preferably, the reaction temperature for the oxidation reaction is 70°C or higher, more preferably 80°C or higher. Generally, temperatures of more than 150°C are not needed, and temperatures below 100°C are preferred e.g. for economic reasons.

As will be understood by the skilled reader, the reaction temperature should not exceed the boiling point of any of the components of the starting material at the pressure at which the reaction is carried out. The reaction can be conveniently carried out at atmospheric pressure, but, if desired, the pressure in the reaction vessel may be higher than atmospheric pressure. In the preferred case where an aqueous medium is present during the oxidation reaction in the process according to the invention, the reaction temperature should thus be less than 100°C if the reaction is carried out at atmospheric pressure. If higher temperatures are desired, the reaction can be carried out at higher pressures.

In order to carry out the oxidation reaction, the starting material comprising the one or more alcohols, the oxoammonium cation or a precursor compound thereof, the co-oxidant and any optional components which may be used in the reaction are combined.

Typically, the starting material comprising the one or more alcohols and the oxoammonium cation or a precursor compound thereof are combined by mixing the starting material with the oxoammonium cation or a precursor compound thereof. This step can be conveniently carried out by mixing the oxoammonium cation or a precursor compound thereof as a solution in an aqueous medium with the starting material. Mixing is typically carried out at an increased temperature, i.e. a temperature as it is discussed as the reaction temperature above. At a temperature where the starting material for the oxidation reaction is in a liquid form (i.e. a molten liquid form), the components of the reaction can be conveniently mixed by stirring. As will be understood, the starting material and the oxoammonium cation or a precursor compound thereof, preferably as a solution in an aqueous medium, can e.g. be combined at a lower temperature, followed by an increase in temperature and mixing. Alternatively, the starting material and the oxoammonium cation or a precursor compound thereof, preferably as a solution in an aqueous medium, can be combined under stirring at an increased temperature.

The co-oxidant can be combined with the starting material comprising the one or more primary alcohols prior to, during or after the starting material has been contacted with the oxoammonium cation or a precursor compound thereof. Also this step can be conveniently carried out by adding and mixing the co-oxidant as a solution in an aqueous medium with the starting material comprising one or more alcohols, or with the starting material and the oxoammonium cation or a precursor compound thereof. Preferably, the co-oxidant is added and mixed as a solution in an aqueous medium with a mixture comprising the starting material and the oxoammonium cation or a precursor compound thereof, typically over a certain period of time, e.g. over 10 min to 5 hours, preferably 30 min to 3 hours. Also the mixing of the co-oxidant with the starting material or with the starting material and the oxoammonium cation or a precursor compound thereof is typically carried out at an increased temperature, i.e. a temperature as it is discussed as the reaction temperature above. Co-oxidant and oxoammonium cation or a precursor compound thereof may also be added simultaneously at once or over a certain period of time, e.g. over 10 min to 5 hours, preferably 30 min to 3 hours.

Other components for the reaction, such as the optional component for the acceleration of the reaction, or additional components of an aqueous medium may be added as needed or as convenient. For example, these components may be contained as a component in an aqueous medium in which the aminoxyl radical is dissolved, and/or in an aqueous medium wherein the co-oxidant is dissolved.

Thus, it will be understood that in accordance with a particularly preferred embodiment, the process in accordance with the present invention comprises
a step of providing a reaction mixture comprising (i) the starting material comprising the one or more primary alcohols, (ii) an aminoxyl radical as a precursor compound for an oxoammonium cation, (iii) a co-oxidant, and (iv) an aqueous medium, by combining the aminoxyl radical with the starting material by mixing the aminoxyl radical as a solution in an aqueous medium with the starting material, and combining the co-oxidant with the starting material by mixing the co-oxidant as a solution in an aqueous medium with a mixture comprising the starting material and the aminoxyl radical, and
allowing the precursor compound to be converted to an oxoammonium cation, and a step of subjecting the starting material to an oxidation reaction using the oxoammonium cation and the co-oxidant as oxidizing agents at a reaction temperature of 50°C or higher, wherein
the starting material comprises one or more primary alcohols of the formula H₃C-(CH₂)ₙ-OH, wherein n + 1 is an integer and is 16 or more, and more preferably, n+1 is 16 or more and 42 or less, still more preferably 20 or more and 40 or less,
the aminoxyl radical is an aminoxyl radical having the structure R₂N-0•, wherein the dot at the oxygen atom indicates an unpaired electron, and wherein the two groups R are independently an organic group bound via a secondary or tertiary carbon atom to the nitrogen, and wherein the two groups R form a heterocyclic ring together with the nitrogen atom to which they are bound, and is more preferably selected from 4-OH-TEMPO, 4-NH₂- TEMPO, 4-MeO-TEMPO, 4-AA-TEMPO, TEMPO, Me-AZADO, ABNO, and from any of these aminoxyl radicals supported on or bound to a carrier,
the co-oxidant is a chlorite,
and optionally a hypochlorite is additionally added prior to the oxidation reaction to which the starting material is subjected.

Once the desired amounts of the oxoammonium cation or its precursor compound, of the co oxidant and of any further optional components have been combined with the starting material, the oxidation reaction is typically allowed to proceed for a certain period of time at the reaction temperature discussed above. The reaction time for the oxidation reaction can be appropriately selected by the skilled person. Typically, it ranges from 1 to 10 hours, preferably from 1 to 5 hours.

As a result of the oxidation reaction, at least a part of the primary alcohols contained in the starting material comprising the one or more primary alcohols is oxidized to the corresponding carboxylic acids. As will be understood by the skilled reader, a carboxylic acid corresponding to a primary alcohol is one wherein the primary alcohol function -CH2-OH is converted to a group -COOH or to a salt thereof. The carbon number of the carboxylic acid is the same as that of the primary alcohol which has undergone the oxidation reaction. Typically, 70 mol% or more, preferably 80 mol% or more, and even more preferably 90 mol% or more of the primary alcohol functions in the starting material (based on the total number of primary alcohol functions as 100 mol%) will be converted to a carboxylic acid group or a salt thereof as a result of the process in accordance with the invention.

Important aspects of the present invention will be summarized in the following items. In this context, it will be appreciated that the first item provides a definition of the process of the invention, whereas the following items define preferred embodiments thereof. Moreover, it should be understood that the following items form part of the present specification, and the information provided above with regard to specific and preferred embodiments continues to apply for these items.
1. A process for the oxidation of alcohols to carboxylic acids, wherein said process comprises:
   a step of subjecting a starting material comprising one or more primary alcohols with a carbon number of 14 or more to an oxidation reaction using an oxoammonium cation and a co-oxidant as oxidizing agents at a reaction temperature of 50°C or higher, to obtain one or more carboxylic acids, wherein the starting material comprising the one or more primary alcohols is a wax or a processed wax resulting from a full or partial hydrolysis of the wax esters contained in the wax before processing.
2. The process of item 1, wherein the one or more primary alcohols are aliphatic primary alcohols.
3. The process of item 1 or 2, wherein the one or more primary alcohols have the formula H₃C-(CH₂)ₙ-OH, wherein n+1 corresponds to the carbon number of the alcohols and is 14 or more.
4. The process of any of items 1 to 3, wherein the starting material comprises one or more primary alcohols having a carbon number of 16 or more.
5. The process of any of items 1 to 4, wherein the starting material comprises one or more primary alcohols selected from primary alcohols with a carbon number of 16 or more and 42 or less, more preferably 20 or more and 40 or less.
6. The process of any of items 1 to 5, wherein the starting material comprises a mixture of primary alcohols as defined in these items with different carbon numbers.
7. The process of item 6, which comprises, prior to the step of subjecting the starting material comprising the one or more primary alcohols to the oxidation reaction, a step of providing the starting material by processing a wax, preferably a natural wax, by a process comprising fully or partially hydrolyzing esters contained therein, and using the processed wax, preferably the processed natural wax, as the starting material for the oxidation reaction.
8. The process of any of items 1 to 7, wherein the oxoammonium cation has the structure R₂N⁺=O, wherein the two groups R are independently an organic group bound via a secondary or tertiary carbon atom to the nitrogen, and wherein the two groups R may be combined to form a heterocyclic ring together with the nitrogen atom to which they are bound.
9. The process of any of items 1 to 8, wherein the oxoammonium cation is selected from a 2,2,6,6-tetramethylpiperidine-oxoammonium cation and from derivatives thereof carrying a substituent at the 4-position of the piperidine ring, from a 9-azabicyclo[3.3.1]nonaneoxoammonium cation, and from a 2-azaadamantane-oxoammonium cation, a 1-methyl-2-azaadamantane-oxoammonium cation and a 1 ,3-dimethyl-2-azaadamantane-oxoammonium cation.
10. The process of any of items 1 to 9, wherein the process comprises:
   a step of providing a reaction mixture comprising (i) the starting material comprising the one or more alcohols, (ii) a precursor compound for an oxoammonium cation, and (iii) a cooxidant, and
   allowing the precursor compound to form an oxoammonium cation.
11. The process of item 10, wherein the precursor compound is selected from an aminoxyl radical or a hydroxylamine.
12. The process of item 10 or 11, wherein the precursor compound is an aminoxyl radical having the structure R2N-0, wherein the dot at the oxygen atom indicates an unpaired electron, and wherein the two groups R are independently an organic group bound via a secondary or tertiary carbon atom to the nitrogen, and wherein the two groups R may form a heterocyclic ring together with the nitrogen atom to which they are bound.
13. The process of item 12, wherein the precursor compound is an aminoxyl radical selected from 2,2,6,6-tetramethylpiperidine-1-oxyl (TEMPO) and from derivatives thereof carrying a substituent at the 4-position of the piperidine ring, from 9-azabicyclo[3.3.1]nonane N-oxyl (ABNO), and from 2-azaadamantane N-oxyl (AZADO), 1-methyl-2-azaadamantane N-oxyl (1-Me-AZADO) and 1,3-dimethyl-2-azaadamantane N-oxyl (1,3-dimethyl-AZADO).
14. The process of item 13, wherein the aminoxyl radical is selected from 4-OH-TEMPO, 4-NH2-TEMPO, 4-MeO-TEMPO, 4-AA-TEMPO, TEMPO, Me-AZADO, and ABNO.
15. The process of item 14, wherein the aminoxyl radical is 4-OH-TEMPO.
16. The process of any of items 10 to 15, wherein the precursor compound is supported on or bound to a carrier.
17. The process of any of items 1 to 16, wherein the oxoammonium cation or the precursor thereof are combined with the starting material comprising one or more alcohols in an amount of 0.1 mol% or more, more preferably 0.2 mol%, and most preferably 0.3 mol% or more, based on the total number of moles of the primary alcohol group in the starting material.
18. The process of any of items 1 to 17, wherein the oxoammonium cation or the precursor thereof is combined with the starting material comprising one or more alcohols in an amount of not more than 20 mol%, more preferably not more than 10 mol%, still more preferably not more than 5 mol% and even more preferably not more than 3 mol%, based on the total number of moles of primary alcohol group in the starting material.
19. The process of any of items 1 to 18, wherein the co-oxidant is a chlorite or a bromite.
20. The process of item 19, wherein the co-oxidant is sodium chlorite.
21. The process of any of items 1 to 20, wherein the co-oxidant is combined with the starting material comprising the one or more primary alcohols in an amount of 0.75 molar equivalents or more, more preferably 1.0 molar equivalents or more, even more preferably 1.05 molar equivalents or more, per mole of primary alcohol group in the starting material.
22. The process of any of items 1 to 21, wherein a hypochlorite is additionally added.
23. The process of any of items 1 to 22, wherein the process is carried out in the absence of an organic solvent.
24. The process of any of items 1 to 23, wherein the oxidation reaction is carried out in the presence of an aqueous medium.
25. The process of item 24, wherein the aqueous medium has a pH of less than 8, more preferably of less than 6.
26. The process of any of items 24 or 25, wherein the aqueous medium comprises a buffer.
27. The process of any of items 24 or 25, wherein the aqueous medium comprises an acid.
28. The process of item 27, wherein the acid is selected from HCl, H₃PO₄, acetic acid and citric acid.
29. The process of any of items 24 to 28, wherein the volume ratio of the organic phase comprising the starting material to be subjected to the oxidation reaction to the aqueous medium ranges from 10:1 to 1:10, more preferably from 5:1 to 1:10, at the reaction temperature of the oxidation reaction.
30. The process of any of items 1 to 29, wherein the reaction temperature of the oxidation reaction is 70°C or more, more preferably 80 °C or more.
31. The process of any of items 1 to 23, wherein the reaction temperature of the oxidation reaction is not higher than 150°C, more preferably less than 100°C.
32. The process of any of items 1 to 31, wherein the reaction temperature of the oxidation reaction is a temperature where the starting material comprising the one or more primary alcohols to be subjected to the oxidation reaction is in a molten liquid form.

### Examples

The following examples further illustrate the process of the present invention.

Analyses: All products were analysed by GC-FID. For pure alcohols the yield of the acid (Y(Acid)) is indicated by its integral ratio over the whole chromatogram (excluding the solvent peak), thereby also representing its purity. For the split rice bran wax (RBW) as a raw material, the conversion of the C₃₀ alcohol to the corresponding C₃₀ acid (X_{C30}-oxidation) is calculated by the division of the integrals representing C₃₀O₂H and the sum of (C₃₀O₂H + C₃₀OH). It is noted that, due to the fact that C₃₀OH and C₂₉O₂H had very similar reference times, these could not easily be differentiated. The amount of C₂₉0_{1/2}H was app. 5 wt.% of the amount of C30O1/2H, therefore only XC3o-oxidations of up to 95 % could be calculated with reasonable analytical effort. The amount of esters in the product was always monitored and determined to be less than 10 % of the RBW product.

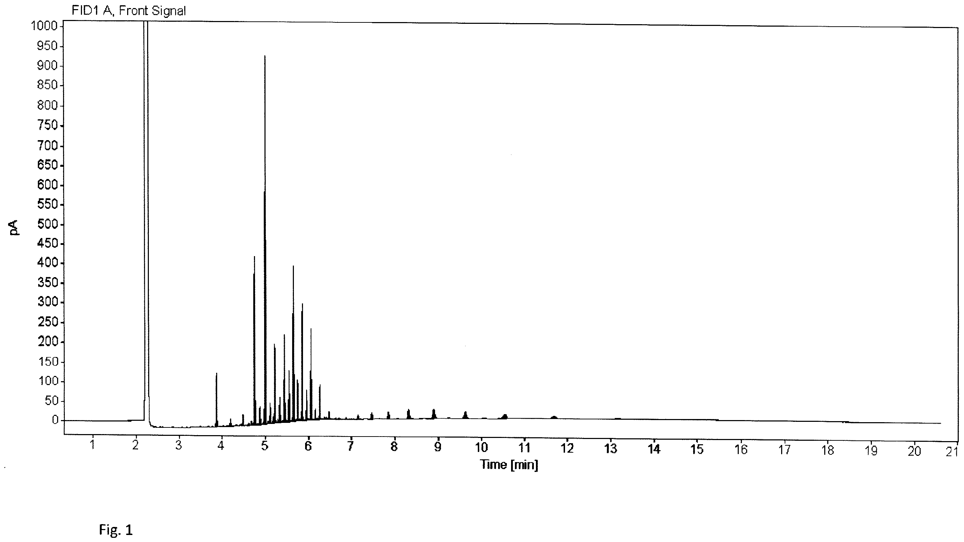

C₃₀-compounds were chosen as representative alcohols/acids for these analyses as these have the medium chain length of the alcohols present, they are the most dominant alcohol species, and close to no free C₃₀ acids exist in unprocessed RBW. An example for its accuracy and reliability can be given by analysing a product of low conversion. Fig. 1 (Chromatogram 1) shows the result of the oxidation with only 0.8 eq. of oxidant. 76 % X_{C30-} oxidation were gained. As can be seen in the chromatogram, all alcohols were oxidized to a similar degree. Materials: For the experiments herein described, chemicals with the usual purity of purchased chemicals were used. NaClO₂ was utilized with 80 % purity, which means that 20 % of the weighed substance was regarded as inactive to the reaction. For NaOCI, the content of free chlorine was reported as specified by the vendor. KOH solutions were prepared with 85 % pure KOH, assuming that the remaining 15 % of the substance were inactive to the reaction. All utilized catalysts and other chemicals were specified with purities of over 95 %.

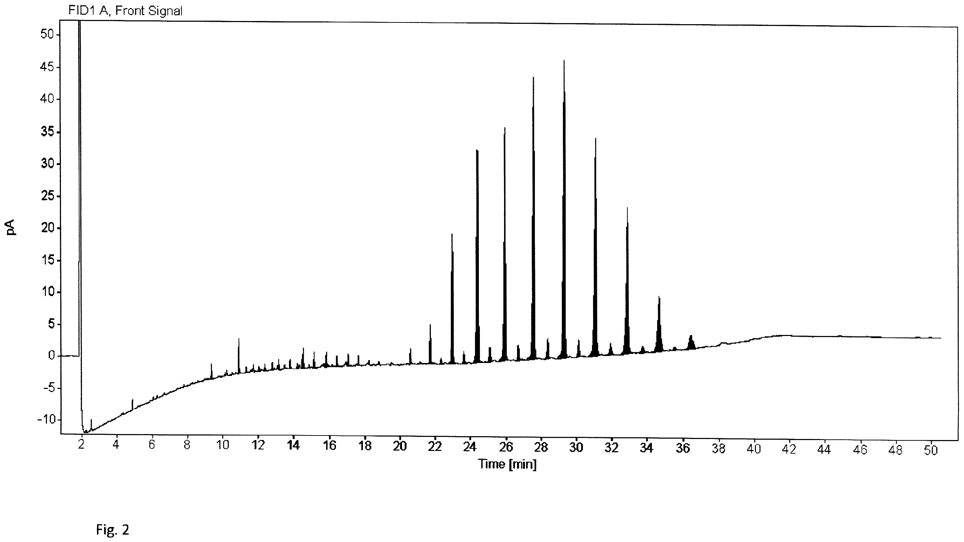

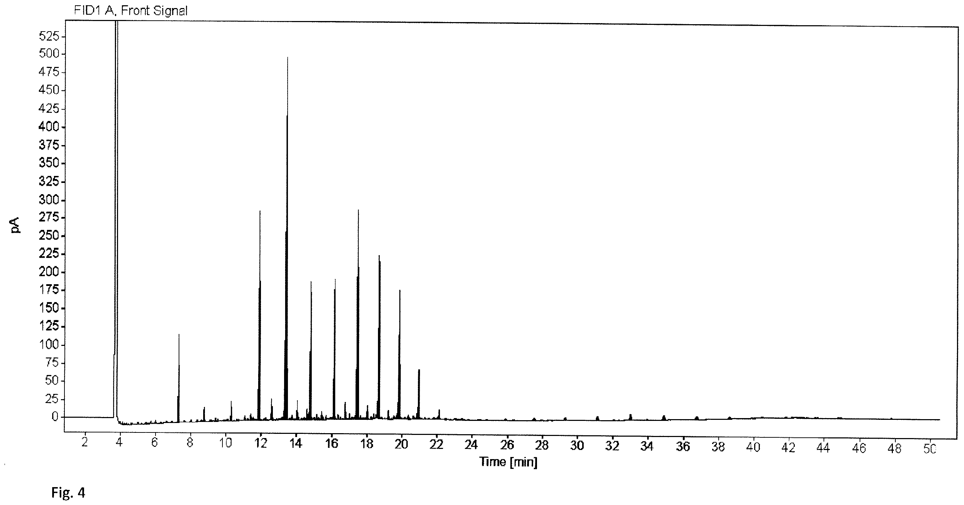

Composition of split Rice Bran Wax (RBW): RBW is an ester wax derived from Rice Bran Oil, which can be gained by extraction of Rice Bran, which is a side product of the dehusking of natural rice to gain white rice. When the esters are hydrolyzed (split), alcohols and acids are obtained. Their composition varies slightly with the origin of the RBW, but will in general consist of equimolar amounts of acids and alcohols (in regards of mass: app. 56.3 wt.% alcohols for the herein utilized RBW). As examples, GC-FID chromatograms of raw, split and oxidized RBW are given (Fig. 2, raw RBW; Fig. 3: Chromatogram 3, alkaline split RBW; Fig. 4: Chromatogram 4, oxidized RBW). For reliable analyses all hydroxyl-groups were trimethylsilylated, which was taken into account unless stated otherwise.

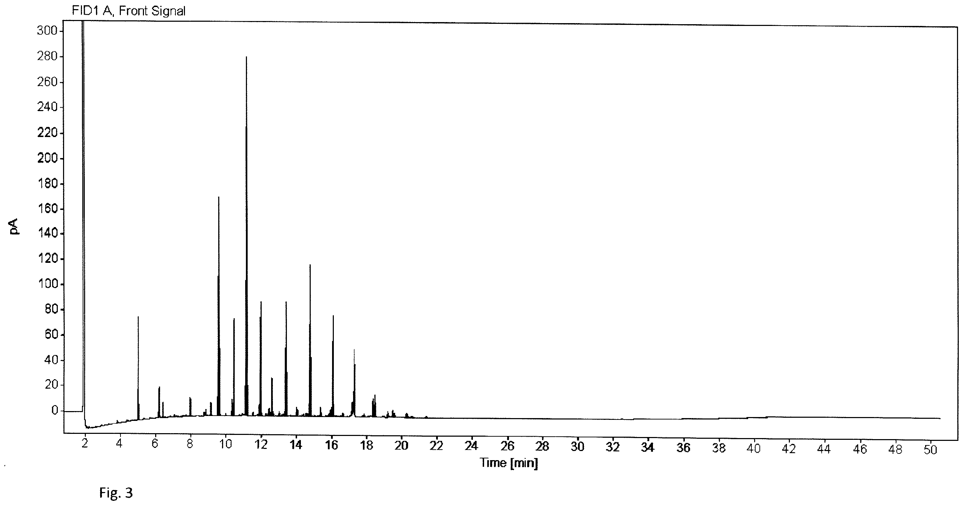

**Table 1: Composition of the major components of hydrolysed Rice Bran Wax (compare Fig. 3, chromatogram 3 regarding the app. retention times). The total area I_{Total} refers to the area calculated by the program, therefore including all major and minor substances besides the solvent. The area ratios are not revised regarding the trimethylsilylation.**

| **Component** | **Retention time [min]** | **Area ratio [I/I_{Total}]** | **Molar contribution (of listed substances)** |
|---|---|---|---|
| | **Acid** | | |
| C₁₆O₂H | 5.0 | 4.3 % | 7.5 % |
| C₂₂O₂H | 9.6 | 10.3% | 13.8% |
| C₂₄O₂H | 11.2 | 22.0% | 27.3% |
| C₂₆O₂H | 12.6 | 1.7% | 1.9% |

| | **Alcohol** | | |
|---|---|---|---|
| C₂₄OH | 10.5 | 3.7 % | 4.5 % |
| C₂₆OH | 12.0 | 4.8 % | 5.6 % |
| C₂₈OH | 13.4 | 7.0 % | 7.6 % |
| C₃₀OH | 14.8 | 13.1 % | 13.3 % |
| C₃₂OH | 16.1 | 9.6 % | 9.2 % |
| C₃₄OH | 17.3 | 7.0 % | 6.3 % |
| C₃₆OH | 18.4 | 2.6 % | 2.2% |
| C₃₈OH | 19.5 | 0.9 % | 0.8 % |
| | | | |
| **Sum** | | 86.8 % | 100.0 % |

Example 1 : 0.2g of C₁₆OH, 93.6 mg NaClO₂, 0.6 mg TEMPO and 0.5 mL H₂O were heated to reflux in a reaction vessel, 10 µL HCl_{(aq., konz.}) were added and after 3 h 98.8 % of the alcohol were converted to C₁₆O₂H.

Procedure 1: The alcohol, additives and catalyst and water are charged at room temperature into the reaction vessel and heated to the 90 °C. If applicable NaOCI is added and subsequently the addition of NaClO₂ dissolved in water is started.

**Table 2: Examples were carried out according to procedure 1.**

| Example | 2 | 3 | 4 |
|---|---|---|---|
| C₁₆OH [g] | 1 | 1 | 1 |
| H₂O (mL) | 2 | 2 | 2 |
| Additive | H₃PO₄ | H₃PO₄ | CA |
| Add. Amount | 1.2mol% | 0.6mol% | 2.1mol% |
| Catalyst | 1.7mol% OH-TEMPO | 0.9mol% AA-TEMPO | 1.9mol% OH-TEMPO |
| NaOCl | 20µL (10% free Cl₂) | 20µL (10% free Cl₂) | - |
| NaClO₂ | 561mg | 561mg | 1.0 eq. |
| H₂O (mL) | 2 | 2 | 1 |
| Time (NaClO₂ addition) | 2h | 2h | 1h |
| Time (stirring, [h]) | 4 | 4 | 2 |
| Y(C₁₆O₂H) | 80.5% | 95.0% | 79.5% |

Procedure 2: 1.0 g split RBW (1.3 mmol alcohols), 5.1 mg citric acid (CA), 2 mL H₂O and the specified catalyst were added to the reaction vessel and heated to 90 °C. 20 µL NaOCI were added before 180 mg NaClO₂ were added in 2 mL H₂O over 2 hours.

**Table 3: Examples were carried out according to Procedure 2.**

| **Example #** | **Catalyst** | **Amount [mol%]** | **X_{C30} [%]** |
|---|---|---|---|
| 5 | OH-TEMPO | 2.2 | 86.5 |
| 6 | NH₂-TEMPO | 2.9 | 77.5 |
| 7 | AA-TEMPO | 2.1 | 94.0 |
| 8 | MeO-TEMPO | 2.7 | 91.0 |
| 9 | Me-AZADO | 1.5 | 94.5 |
| 10 | ABNO | 2.7 | 77.5 |
| 11 | TEMPO on SiO₂ | -2.2 | 55.0^{E} |
| 12 | TEMPO polymer-bound | -3.1 | 57.0^{E} |
| | ^{E} Ester formation >10% was observed in these cases. | | |

Procedure 3: 1.0 g split RBW and 3 mL 1 m HOAc/NaOAc buffer were added into the reaction vessel and heated to 90 °C. 20 µL NaOCI was added, before the specified amount of NaClO₂ and 2.0 mg TEMPO were added in 2.2 mL of H₂O via syringe pump over 2 hours.

**Table 4: Examples were carried out according to Procedure 3.**

| **Experiment #** | **NaClO₂ [eq.]** | **X_{C30} [%]** |
|---|---|---|
| 13 | 0.80 | 76.0 |
| 14 | 0.90 | 80.5 |
| 15 | 1.00 | 89.0 |
| 16 | 1.09 | 91.0 |
| 17 | 1.10 | 93.5 |
| 18 | 1.20 | 94.5 |
| 19 | 1.30 | 94.5 |
| 20 | 1.41 | 94.0 |

Procedure 4: 0.50 g C₁₆OH and 1 mL of buffer medium with the specified reagents were added into the reaction vessel, heated to 95 °C and the reaction was started by addition of 20 µL NaOCI, 1.3 eq. of NaClO₂(aq.) (0.24g/mL) were added within 60 min by pipetting.

**Table 5: Examples according to Procedure 4.**

| | **TEMPO** [**mol%]** | **HOAc [mmol]** | **NaOAc [mmol]** | **Additive** | **Y(C₁₆O₂H)** |
|---|---|---|---|---|---|
| 21 | 0.76 | 0.25 | 0.25 | | 98.0% |
| 22 | 0.86* | 0.01 | 0.09 | 250mg Aceton | 64.0% |
| 23 | 0.94* | 0.5 | 0.5 | | 87.5% |

| | | | | | |
|---|---|---|---|---|---|
| * No NaOCI added, NaClO₂ added within 25 min. | | | | | |

Procedure 5: 1.0 g split RBW (1.3 mmol alcohols) and 3 mL 1 m HOAc/NaOAc buffer were added into the reaction vessel and heated to 90 °C. 20 µL NaOCI were added, before 225 mg NaClO₂ and the specified amount of TEMPO were added in 2 mL of H₂O via syringe pump over 2 hours.

**Table 1: Examples according to Procedure 5.**

| | **TEMPO [mol%]** | **X_{C30} [%]** |
|---|---|---|
| 24 | 0.25 | 74.0 |
| 25 | 0.49 | 86.0 |
| 26 | 0.75 | 93.0 |
| 27 | 0.99 | 94.0 |

Procedure 6: 1.0 g split RBW (1.3 mmol alcohols) and 3 mL 1 m HOAc/NaOAc buffer and the specified amount of TEMPO were added into the reaction vessel and heated to 90 °C. 20 µL NaOCI were added, before 225 mg NaClO₂ was added in 2 mL of H₂O via syringe pump over 1 hour.

**Table 2: Examples according to Procedure 6.**

| | **TEMPO [mol%]** | **X_{C30} [%]** |
|---|---|---|
| 28 | 0.05 | 17.5 |
| 29 | 0.25 | 58.0 |
| 30 | 0.49 | 88.5 |
| 31 | 0.98 | 94.0 |

Procedure 7: 1.5 g split RBW (1.9 mmol alcohols), 7.5 mg CA, 10.1 mg OH-TEMPO and the specified amount of water were added to the reaction vessel and heated to 90 °C. 30 µL NaOCI was added before 270 mg NaClO₂ were added in 0.75 mL H₂O over 2 hours.

**Table 8: Examples according to Procedure 7.**

| | **H₂O [mL]** | **X_{C30} [%]** |
|---|---|---|
| 32 | 0.25 | 93.8 |
| 33 | 0.50 | 93.8 |
| 34 | 0.75 | 94.4 |
| 35 | 1.5 | 94.4 |

Procedure 8: 2.0 g split RBW (2.6 mmol alcohols), 10.0 mg OH-TEMPO and 2 mL aqueous buffer provided by KOH and citric acid ("CA") as indicted in the table below were added into the reaction vessel and heated to 90 °C. 40 µL NaOCl were added prior to the addition of 370 mg NaClO₂ in 1 mL H₂O over 2 hours.

**Table 9: Examples according to Procedure 8.**

| | **KOH [eq.]** | **CA [eq.]** | **X_{C30} [%]** |
|---|---|---|---|
| 36 | 1.3 | 0.44 | 89.7 |
| 37 | 1.3 | 0.52 | 93.5 |
| 38 | 1.3 | 0.65 | 93.3 |
| 39 | 1.5 | 0.75 | 92.8 |

Example 40: 1.0 g split RBW (1.3 mmol alcohols), 1.3 mol% OH-TEMPO and 1 mL aqueous buffer consisting of 182 mg K_{2.5}H_{0.5}(CA) were added into the reaction vessel and heated to 90 °C. 15 µL NaOCl were added prior to the addition of 315 mg NaClO₂ in 1 mL H₂O over 15 minutes. A C₃₀-oxidation of 93.5 % was attained, accompanied by only 4.0 % esters.

Procedure 9: The specified amount of split RBW, additives and catalyst are set in an adequate reaction vessel, heated to the 90 °C and stirred with an over-head stirrer. If applicable NaOCI is added and subsequently the addition of NaClO₂ dissolved in water is started.

**Table 10: Examples according to Procedure 9.**

| Example # | 41 | 42 | 43 |
|---|---|---|---|
| RBW (g) | 20 | 500 | 1000 |
| H₂O (mL) | 65 | 920 | 990 |
| Additive | 0.15 mol Na_{0.33}H_{0.66}OAc | 13mmol CA | 0.69mol K_{2.5}H_{0.5}(CA) |
| Catalyst | 36.5mg TEMPO | 3 .0g OH-TEMPO | 6.0 g OH-TEMPO |
| NaOCl | 0.2g (5% free Cl₂) | 12.4g (5% free Cl₂)) | 40g (5% free Cl₂)) |
| NaClO₂ (g) | 4.5 | 90 | 180 |
| H₂O for NaClO₂ (mL) | 20 | 300 | 300 |
| Time (NaClO₂ addition) | 20min | 2.5h | 2.5h |
| Time (stirring) | 80min | 12h | 1.5h |
| C₃₀-oxidation | 92% | 90% | > 94 % |
| Acid number (AN) | 130 | 114 | 132 |

Example 44: 1.0 g split RBW, 8.5 mg citric acid, 2 mL H₂O and 73.2 mg TINUVIN NOR 371 FF (a hydroxylamine precursor compound for an oxoammonium cation carrying a groups NOR immobilized on a polymer) were put in a reaction vessel and heated to 90 °C. 40 µL NaOCI were added, before 183 mg NaClO₂, dissolved in app. 1.2 mL H₂O were added within 80 minutes. After 6 h of stirring a X₃₀ conversion of 93.5 % was attained.

## Claims

1. A process for the oxidation of alcohols to carboxylic acids, wherein said process comprises:
a step of subjecting a starting material comprising one or more primary alcohols with a carbon number of 14 or more to an oxidation reaction using an oxoammonium cation and a co-oxidant as oxidizing agents at a reaction temperature of 50°C or higher, to obtain one or more carboxylic acids, wherein the starting material comprising the one or more primary alcohols is a wax or a processed wax resulting from a full or partial hydrolysis of the wax esters contained in the wax before processing.

2. The process of claim 1, wherein the starting material comprises one or more primary alcohols selected from primary alcohols with a carbon number of 16 or more and 42 or less.

3. The process of claim 1 or 2, wherein the oxoammonium cation has the structure R₂N⁺=O, wherein the two groups R are independently an organic group bound via a secondary or tertiary carbon atom to the nitrogen, and wherein the two groups R may be combined to form a heterocyclic ring together with the nitrogen atom to which they are bound.

4. The process of any of claims 1 to 3, wherein the oxoammonium cation is selected from a 2,2,6,6-tetramethylpiperidine-oxoammonium cation and from derivatives thereof carrying a substituent at the 4-position of the piperidine ring, from a 9-azabicyclo[3.3.1]nonane-oxoammonium cation, and from a 2-azaadamantane-oxoammonium cation, a 1-methyl-2-azaadamantane-oxoammonium cation and a 1,3-dimethyl-2- azaadamantane-oxoammonium cation.

5. The process of any of claims 1 to 4, wherein the process comprises:
a step of providing a reaction mixture comprising (i) the starting material comprising the one or more alcohols, (ii) a precursor compound for an oxoammonium cation, and (iii) a cooxidant, and
allowing the precursor compound to form an oxoammonium cation.

6. The process of claim 5, wherein the precursor compound is selected from an aminoxyl radical or a hydroxylamine.

7. The process of any of claims 1 to 6, wherein the oxoammonium cation or the precursor thereof are combined with the starting material comprising one or more alcohols in an amount of 0.1 mol% or more, based on the total number of moles of the primary alcohol group in the starting material.

8. The process of any of claims 1 to 7, wherein the co-oxidant is a chlorite or a bromite.

9. The process of any of claims 1 to 8, wherein the co-oxidant is combined with the starting material comprising the one or more primary alcohols in an amount of 0.75 molar equivalents or more, more preferably 1.0 molar equivalents or more, even more preferably 1.05 molar equivalents or more, per mole of primary alcohol group in the starting material.

10. The process of any of claims 1 to 9, wherein a hypochlorite is additionally added.

11. The process of any of claims 1 to 10, wherein the process is carried out in the absence of an organic solvent.

12. The process of any of claims 1 to 11, wherein the oxidation reaction is carried out in the presence of an aqueous medium.

13. The process of claim 12, wherein the volume ratio of the organic phase comprising the starting material to be subjected to the oxidation reaction to the aqueous medium ranges from 10:1 to 1:10, more preferably from 5:1 to 1:10, at the reaction temperature of the oxidation reaction.

14. The process of any of claims 1 to 13, wherein the reaction temperature of the oxidation reaction is a temperature where the starting material comprising the one or more primary alcohols to be subjected to the oxidation reaction is in a molten liquid form.

## Patentansprüche

1. Verfahren zur Oxidation von Alkoholen zu Carbonsäuren, wobei das Verfahren Folgendes umfasst:
einen Schritt des Unterwerfens eines Ausgangsmaterials, das einen oder mehrere primäre Alkohole mit einer Kohlenstoffanzahl von 14 oder mehr umfasst, einer Oxidationsreaktion unter Verwendung eines Oxoammoniumkations und eines Co-Oxidationsmittels als Oxidationsmittel bei einer Reaktionstemperatur von 50°C oder höher, um eine oder mehrere Carbonsäuren zu erhalten, wobei das Ausgangsmaterial, das den einen oder die mehreren primären Alkohole umfasst, ein Wachs oder ein verarbeitetes Wachs ist, das aus einer vollständigen oder teilweisen Hydrolyse der vor der Verarbeitung in dem Wachs enthaltenen Wachsester resultiert.

2. Verfahren nach Anspruch 1, wobei das Ausgangsmaterial einen oder mehrere primäre Alkohole umfasst, ausgewählt aus primären Alkoholen mit einer Kohlenstoffanzahl von 16 oder mehr und 42 oder weniger.

3. Verfahren nach Anspruch 1 oder 2, wobei das Oxoammoniumkation die Struktur R₂N⁺=O hat, wobei die beiden Gruppen R unabhängig voneinander eine organische Gruppe sind, die über ein sekundäres oder tertiäres Kohlenstoffatom an den Stickstoff gebunden ist, und wobei die beiden Gruppen R zusammen mit dem Stickstoffatom, an das sie gebunden sind, einen heterocyclischen Ring bilden können.

4. Verfahren nach einem der Ansprüche 1 bis 3, wobei das Oxoammoniumkation ausgewählt ist aus einem 2,2,6,6-Tetramethylpiperidin-Oxoammoniumkation und aus Derivaten davon, die einen Substituenten in der 4-Position des Piperidinrings tragen, aus einem 9-Azabicyclo[3.3.1]nonan-Oxoammoniumkation, und aus einem 2-Azaadamantan-Oxoammoniumkation, einem 1-Methyl-2-azaadamantan-Oxoammoniumkation und einem 1,3-Dimethyl-2-azaadamantan-Oxoammoniumkation.

5. Verfahren nach einem der Ansprüche 1 bis 4, wobei das Verfahren Folgendes umfasst:
einen Schritt des Bereitstellens einer Reaktionsmischung, umfassend (i) das Ausgangsmaterial, das den einen oder die mehreren Alkohole umfasst, (ii) eine Vorläuferverbindung für ein Oxoammoniumkation und (iii) ein Co-Oxidationsmittel, und
Ermöglichen, dass die Vorläuferverbindung ein Oxoammoniumkation bildet.

6. Verfahren nach Anspruch 5, wobei die Vorläuferverbindung ausgewählt ist aus einem Aminoxylrest oder einem Hydroxylamin.

7. Verfahren nach einem der Ansprüche 1 bis 6, wobei das Oxoammoniumkation oder der Vorläufer davon mit dem Ausgangsmaterial kombiniert werden, das einen oder mehrere Alkohole in einer Menge von 0,1 Mol-% oder mehr, bezogen auf die Gesamtmolzahl der primären Alkoholgruppe in dem Ausgangsmaterial, umfasst.

8. Verfahren nach einem der Ansprüche 1 bis 7, wobei das Co-Oxidationsmittel ein Chlorit oder ein Bromit ist.

9. Verfahren nach einem der Ansprüche 1 bis 8, wobei das Co-Oxidationsmittel mit dem Ausgangsmaterial kombiniert wird, das den einen oder die mehreren primären Alkohole in einer Menge von 0,75 molaren Äquivalenten oder mehr, bevorzugter 1,0 molaren Äquivalenten oder mehr, noch bevorzugter 1,05 molaren Äquivalenten oder mehr, pro Mol der primären Alkoholgruppe in dem Ausgangsmaterial umfasst.

10. Verfahren nach einem der Ansprüche 1 bis 9, wobei zusätzlich ein Hypochlorit zugesetzt wird.

11. Verfahren nach einem der Ansprüche 1 bis 10, wobei das Verfahren in Abwesenheit eines organischen Lösungsmittels durchgeführt wird.

12. Verfahren nach einem der Ansprüche 1 bis 11, wobei die Oxidationsreaktion in Gegenwart eines wässrigen Mediums durchgeführt wird.

13. Verfahren nach Anspruch 12, wobei das Volumenverhältnis der organischen Phase, die das der Oxidationsreaktion zu unterziehende Ausgangsmaterial umfasst, zu dem wässrigen Medium bei der Reaktionstemperatur der Oxidationsreaktion im Bereich von 10:1 bis 1:10, bevorzugter von 5:1 bis 1:10, liegt.

14. Verfahren nach einem der Ansprüche 1 bis 13, wobei die Reaktionstemperatur der Oxidationsreaktion eine Temperatur ist, bei der das Ausgangsmaterial, das den einen oder die mehreren primären Alkohole umfasst, die der Oxidationsreaktion unterworfen werden sollen, in einer geschmolzenen flüssigen Form vorliegt.

## Revendications

1. Procédé pour l'oxydation d'alcools en acides carboxyliques, dans lequel ledit procédé comprend :
une étape consistant à soumettre un produit de départ comprenant un ou plusieurs alcools primaires ayant un nombre d'atomes de carbone de 14 ou plus à une réaction d'oxydation en utilisant en tant qu'agents oxydants un cation oxoammonium et un co-oxydant, à une température de réaction de 50 °C ou plus, pour obtenir un ou plusieurs acides carboxyliques, dans lequel le produit de départ comprenant lesdits un ou plusieurs alcools primaires est une cire ou une cire traitée résultant d'une hydrolyse partielle ou totale des esters de cire contenus dans la cire avant le traitement.

2. Procédé selon la revendication 1, dans lequel le produit de départ comprend un ou plusieurs alcools primaires choisis parmi les alcools primaires ayant un nombre d'atomes de carbone de 16 ou plus et 42 ou moins.

3. Procédé selon la revendication 1 ou 2, dans lequel le cation oxoammonium a la structure R₂N⁺=O, dans laquelle les deux groupes R représentent indépendamment un groupe organique lié à l'atome d'azote par un atome de carbone secondaire ou tertiaire, et dans laquelle les deux groupes R peuvent être combinés pour former un cycle hétérocyclique conjointement avec l'atome d'azote auquel ils sont liés.

4. Procédé selon l'une quelconque des revendications 1 à 3, dans lequel le cation oxoammonium est choisi parmi un cation 2,2,6,6-tétraméthylpipéridine-oxoammonium et parmi ses dérivés portant un substituant à la position 4 du cycle pipéridine, parmi un cation 9-azabicyclo[3.3.1]nonane-oxoammonium, et parmi un cation 2-azaadamentane-oxoammonium, un cation 1-méthyl-2-azaadamentane-oxoammonium et un cation 1,3-diméthyl-2-azaadamentane-oxoammonium.

5. Procédé selon l'une quelconque des revendications 1 à 4, dans lequel le procédé comprend :
une étape consistant à fournir un mélange réactionnel comprenant (i) le produit de départ comprenant lesdits un ou plusieurs alcools, (ii) un composé précurseur pour un cation oxoammonium, et (iii) un co-oxydant, et
permettre au composé précurseur de former un cation oxoammonium.

6. Procédé selon la revendication 5, dans lequel le composé précurseur est choisi parmi un radical aminoxyle ou une hydroxylamine.

7. Procédé selon l'une quelconque des revendications 1 à 6, dans lequel le cation oxoammonium ou son précurseur est combiné avec le produit de départ comprenant un ou plusieurs alcools, en une quantité de 0,1 % en moles ou plus, par rapport au nombre total de moles du groupe alcool primaire dans le produit de départ.

8. Procédé selon l'une quelconque des revendications 1 à 7, dans lequel le co-oxydant est un chlorite ou un bromite.

9. Procédé selon l'une quelconque des revendications 1 à 8, dans lequel le co-oxydant est combiné avec le produit de départ comprenant lesdits un ou plusieurs alcools primaires, en une quantité de 0,75 équivalent molaire ou plus, de façon plus particulièrement préférée 1,0 équivalent molaire ou plus, de façon encore plus particulièrement préférée 1,05 équivalent molaire ou plus, par mole de groupe alcool primaire dans le produit de départ.

10. Procédé selon l'une quelconque des revendications 1 à 9, dans lequel un hypochlorite est en outre ajouté.

11. Procédé selon l'une quelconque des revendications 1 à 10, dans lequel le procédé est effectué en l'absence d'un solvant organique.

12. Procédé selon l'une quelconque des revendications 1 à 11, dans lequel la réaction d'oxydation est effectuée en la présence d'un milieu aqueux.

13. Procédé selon la revendication 12, dans lequel le rapport en volume de la phase organique comprenant le produit de départ destiné à être soumis à la réaction d'oxydation au milieu aqueux se situe dans la plage allant de 10:1 à 1:10, de façon plus particulièrement préférée de 5:1 à 1:10, à la température de réaction de la réaction d'oxydation.

14. Procédé selon l'une quelconque des revendications 1 à 13, dans lequel la température de réaction de la réaction d'oxydation est une température à laquelle le produit de départ comprenant lesdits un ou plusieurs alcools primaires destinés à être soumis à la réaction d'oxydation se trouve sous une forme liquide fondue.
